# EUROPÄISCHE PATENTSCHRIFT

(11) **EP 0 889 049 B1**
(45) Veröffentlichungstag und Bekanntmachung des Hinweises auf die Patenterteilung: **16.04.2003**
(21) Anmeldenummer: 98111705.4
(22) Anmeldetag: 25.06.1998
(51) Int. Cl.: C07F 9/74, C07F 9/80, C07F 15/00, C07D 405/04, C07B 53/00

(54) **Neue Diarsinverbindungen**
Novel diarsine compounds
Nouveaux composés de diarsines

(30) Priorität: 02.07.1997 EP 97110940
(43) Veröffentlichungstag der Anmeldung: 07.01.1999
(73) Patentinhaber: F. HOFFMANN-LA ROCHE AG, 4070 Basel (CH)
(72) Erfinder: Broger, Emil Albin, 4312 Magden (CH); Cereghetti, Marco, 4058 Basel (CH); Kienzle, Frank, 4113 Flüh (CH)
(74) Vertreter: Braun, Axel

(56) Entgegenhaltungen:
- EP-A- 0 398 132
- KOJIMA A ET AL: "Synthesis and Evaluation of a New Chiral Arsine Ligand;2,2 ? -bis(diphenylarsino)-1,1 ?-binaphthyl (BINAs)" TETRAHEDRON LETTERS, Bd. 38, Nr. 19, 12. Mai 1997, Seite 3459-3460 XP004061453
- SUK Y C ET AL: "Synthesis and Evaluation of a New Chiral Ligand: 2-diphenylarsino -2 ?-diphenylphosphino-1,1 ?-binaphthyl (BINAPAs)" TETRAHEDRON LETTERS, Bd. 39, Nr. 13, 26. März 1998, Seite 1773-1776 XP004108473
- D. W. ALLEN: "Synthesis and reactions of 2,2'-biphenylylenebisdiethylphosphine." JOURNAL OF THE CHEMICAL SOCIETY, SECTION C: ORGANIC CHEMISTRY.,1967, Seiten 1869-1875, XP002077991 LETCHWORTH GB

## Beschreibung

Die vorliegende Erfindung betrifft neue, chirale, in (R)- oder (S)-Form oder als Racemat vorliegende Diarsinverbindungen der allgemeinen Formel 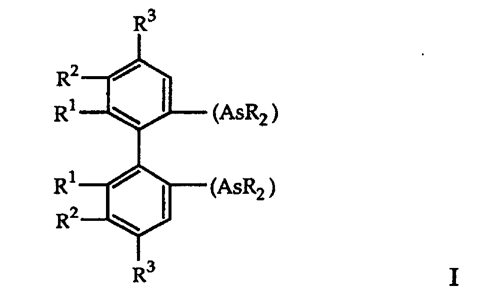 worin
- R: ein gegebenenfalls substituiertes Aryl aus der Gruppe von Phenyl, Naphthyl, Furyl und Thienyl; C₃₋₈-Cycloalkyl oder C₁₋₈-Alkyl;
- R¹ Methyl, R², R³: jeweils unabhängig voneinander C₁₋₈-Alkyl, C₁₋₈-Alkoxy, Aryloxy, F oder Cl,
- R² und R³: unabhängig voneinander auch Wasserstoff oder
- R¹ und R²: zusammen Tetramethylen, Dioxomethylen oder ein Benzo- oder Benzofuro- System an dem jeweiligen Benzolring bedeuten.

Die Erfindung betrifft ferner die Herstellung der Diarsinverbindungen gemäss der allgemeinen Formel I, sowie Komplexe der Verbindungen der allgemeinen Formel I mit Metallen der Gruppe VIII. Weiterhin betrifft die vorliegende Erfindung die Verwendung der Diarsinverbindung der Formel I, bzw. der chiralen in der (R)- oder (S)-Form oder als Racemat vorliegenden Darsinverbindungen der Formel I worin
- R: ein gegebenenfalls substituiertes Aryl aus der Gruppe von Phenyl, Naphthyl, Furyl und Thienyl; C₃₋₈-Cycloalkyl oder C₁₋₈-Alkyl;
- R¹ R², R³,: jeweils unabhängig voneinander C₁₋₈-Alkyl, C₁₋₈- Alkoxy, Aryloxy, F oder Cl,
- R² und R³: unabhängig voneinander auch Wasserstoff oder
- R¹ und R²: zusammen Tetramethylen, Dioxomethylen oderein Benzofuro-System an dem jeweiligen Benzolring bedeuten,
in Form ihrer Komplexe mit einem Metall der Gruppe VIII, als Katalysatoren für enantioselektive Reaktionen, wie z.B. asymmetrischen Hydrierungen (von z.B. Chromenylpyridin-N-oxiden und Chromenylpyridium), enatioselektive Wasserstoffverschiebungen und dergleichen.

EP-A-0398132 beschreibt Diphosphorverbindungen, deren Herstellung, diese Liganden enthaltene Metallkomplexe (Metall z.B. Rhodium) und die Verwendung der Komplexe als Katalysatoren für asymmetrische Hydrierungen.

Tetrahedron Letters, Bd. 8, Nr. 19, Seiten 3456 - 3460 beschreibt, dass zu üblichen Phosphorliganden analoge Arsenligen in der asymmetrischen Heck-Reaktion eingesetzt werden können.

Unter den bekannten Arsinverbindungen sind nur wenige chirale Verbindungen bekannt, die als Liganden in metallkatalysierten asymmetrischen Reaktionen Anwendung finden (H.B. Kagan, Asymmetric Synthesis, Vol 5, Ed. J.D. Morrison, Academic Press). Unter Verwendung dieser bekannten Arsinverbindungen in Metallkomplexen bei asymmetrischen Reaktionen liegen die optischen Ausbeuten im Bereich von 27%.

Aufgabe der vorliegenden Erfindung ist es neue chirale Diarsinverbindungen zur Verfügung zu stellen, die zudem in enantioselektiven Reaktionen Anwendung finden und bessere optische Ausbeuten ermöglichen.

Die Aufgabe wird durch die erfindungsgemässen atropisomeren Diarsinverbindungen der Formel I gelöst.

Der Ausdruck "C₁₋₈-Alkyl" bedeutet im Rahmen der vorliegenden Erfindung geradkettige oder verzweigte Alkylgruppen mit 1-8 Kohlenstoffatomen, wie z.B. Methyl, Ethyl, Propyl, Isopropyl, Butyl, Isobutyl, tert.Butyl, Pentyl, Isopentyl, Neopentyl, Hexyl, Isohexyl, Heptyl oder Octyl. Der Ausdruck "C₁₋₈-Alkoxy" bedeutet Ethergruppen, in denen der Alkylrest wie oben definiert ist.

Der Ausdruck "C₃₋₈-Cycloalkyl" bedeutet in der vorliegenden Erfindung 3-8-gliedrige Ringe, wie z.B. Cyclopropyl, Cyclobutyl, Cyclopentyl, Cyclohexyl, Cycloheptyl und Cyclooctyl, welche gegebenenfalls mit Alkyl-, Alkoxy- oder Arylgruppen substituiert sein können

Der Ausdruck "gegebenenfalls substituiertes Aryl", bedeutet im Rahmen der vorliegenden Erfindung Phenyl, Naphthyl, Furyl und Thienyl-Reste, welche unsubstituiert oder einfach oder mehrfach substituiert sein können. Als Substituenten kommen z.B. Phenyl, C₁₋₈-Alkyl- oder C₁₋₈-Alkoxygruppen, Di-C₁₋₆-Alkylamine und Halogene in Frage.

Der Ausdruck "Aryloxy" bedeutet Ethergruppen, in denen Aryl wie oben definiert ist.

Der Ausdruck "Benzofuro- System" bezeichnet die Reste 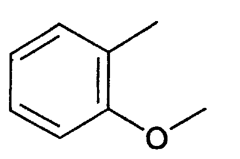 welche auch durch C₁₋₈-Alkyl- oder C₁₋₈-Alkoxy oder Phenyl substituiert sein können.

Besonders bevorzugt sind Diarsinverbindungen in denen R¹ Methyl; R² und R³ Wasserstoff und R Phenyl, oder R¹ und R² zusammen Benzo und R³ Wasserstoff, ist.

Ganz besonders bevorzugt sind optisch aktive Verbindungen der Formel I, wie beispielsweise
(R) bzw.(S) -(6,6'-Dimethylbiphenyl-2,2'-diyl)bis(diphenylarsin)
(R) bzw. (S)-(6,6'-Dimethylbiphenyl-2,2'-diyl)bis(dicyclohexylarsin)
(R) bzw. (S)-(6,6'-Dimethylbiphenyl-2,2'-diyl)bis(di-p-tolylarsin).

Die erfindungsgemässen Diarsinverbindungen der Formel I bilden Komplexe mit Uebergangsmetallen der Gruppe VIII, insbesondere mit Ruthenium und Rhodium, welche als Katalysatoren bei asymmetrischen Hydrierungen und auch für enantioselektive Wasserstoffverschiebungen verwendbar sind. Für die erwähnten Hydrierungen sind Rhodium- und Ruthenium-, und Iridium-Komplexe bevorzugt. Diese Katalysatoren, d.h. die Komplexe aus einem Metall der Gruppe VIII und den Diarsinverbindungen der Formel I sind neu und ebenfalls Gegenstand der vorliegenden Erfindung.

Beispiele solcher optisch aktiven Metall-Komplexe sind insbesondere optisch aktive kationische und neutrale Rhodium- und Ruthenium-Komplexe der allgemeinen Formel II-a bis II-e

[Rh(Y)(Lₙ)]⁺A⁻ II-a

[Rh(Y)(Lₙ)B] II-b

[Ru(Y)]²⁺(A⁻)₂ II-c

[Ru(Y)(B)₂] II-d

[Ru(Y)(C¹)(C²)₂₋ₘ](C³)ₘ II-e

worin
- L: neutraler Ligand
- A: Anion einer Sauerstoffsäure oder Komplexsäure
- B: anionisch koordinierender Ligand
- C¹: Benzol, p-Cymol, Xylol, Hexamethylbenzol
- C²: Halogen
- C³: Halogen oder A
- n: 0, 1 oder 2
- m: 0, 1 oder 2
- Y: ein chirales atropisomeres Diarsin der Formel I bedeuten.

Der Ausdruck "anionisch koordinierender Ligand" umfasst z.B. Halogen, einen Carbonsäurerest, einen Sulfonatrest wie z.B. Tosylat oder Methansulfonat, ein 1,3-Diketonat wie z.B. Acetylacetonat, ein gegebenenfalls substituiertes Phenolat, Hydroxy, Nitrat, Nitrit, Cyanat, Rhodanid, Cyanid, Allyl und 2-Methylallyl.

Der Ausdruck "neutraler Ligand" bedeutet im Rahmen der vorliegenden Erfindung einen austauschbaren Liganden, z.B. Olefine wie Ethylen, Propylen, Cycloocten, 1,5-Hexadien, Norbornadien, 1,5-Cyclooctadien, und dergleichen, Nitrile wie Acetonitril, Benzonitril, oder auch das verwendete Lösemittel. Dieser Ligand kann bei der Hydrierung ausgetauscht werden. Falls mehr als ein solcher Ligand vorhanden ist, können diese auch voneinander verschieden sein.

Der Ausdruck "Sauerstoffsäure oder Komplexsäure" bedeutet im Rahmen der vorliegenden Erfindung Säuren aus der Gruppe von H₂SO₄, HClO₄, HBrO₄, HIO₄, HNO₃, H₃PO₄, H₃PO₃, CF₃SO₃H sowie Halogenkomplexe mit den Elementen Bor, Phosphor, Arsen, Antimon oder Bismut. Bevorzugte Vertreter sind HClO₄, CF₃SO₃H, HPF₆, HBF₄, HB(Ph)₄, HB(3,5(CF₃)₂-C₆H₃)₄, HSbF₆ und HAsF₆.

Der Ausdruck "Halogen" umfasst Fluor, Chlor, Brom und Iod.

Das Verfahren zur Herstellung der erfindungsgemässen Diarsinverbindungen der allgemeinen Formel I ist dadurch gekennzeichnet, dass man eine Verbindung der allgemeinen Formel III 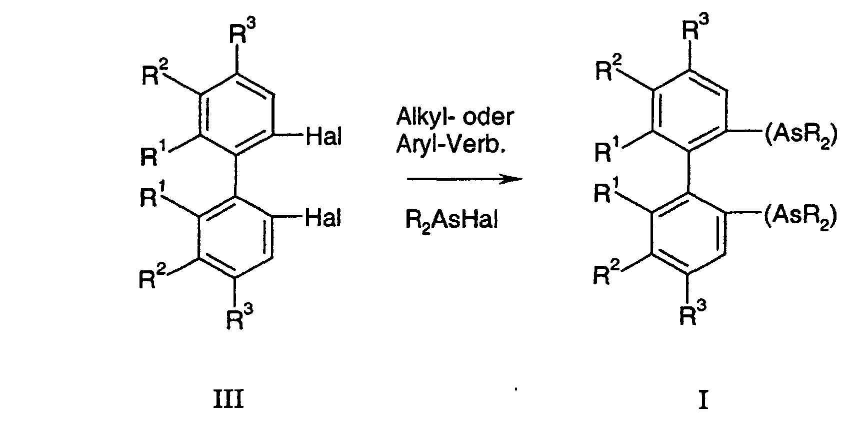 worin
- R¹: Methyl,
- R², R³: jeweils unabhängig voneinander C₁₋₈-Alkyl, C₁₋₈-Alkoxy, Aryloxy, F oder Cl,
- R² und R³: unabhängig voneinander auch Wasserstoff oder
- R¹ und R²: zusammen Tetramethylen, Dioxomethylen oder ein Benzo- oder Benzofuro- System an dem jeweiligen Benzolring bedeuten,
mit einer Verbindung der Formel R₂As Hal, worin
- R: ein gegebenenfalls substituiertes Aryl aus der Gruppe von Phenyl, Naphthyl, Furyl und Thienyl; C₃₋₈-Cycloalkyl oder C₁₋₈-Alkyl
bedeutet und Hal Brom oder Iod bedeutet, in Gegenwart einer Alkali-alkyl- bzw. einer Alkali-aryl-Verbindung reagieren lässt.

Der Ausdruck "Alkali" umfasst die Alkalimetalle Lithium, Natrium und Kalium, wobei Lithium bevorzugt ist.

Als Lösemittel werden aliphatische Kohlenwasserstoffe wie Pentan, Hexan, Heptan, Oktan und Isomere davon; oder aromatische Kohlenwasserstoffe wie Benzol, Toluol, Xylol, Tetrahydrofuran oder ähnliche; und/oder Ether wie Dimethylether, Diethylether, Diisopropylether oder ähnliche verwendet. Bevorzugt werden Aromat-Ethergemische wie z.B. Toluol/Diethylether verwendet.

Die Verbindungen R₂AsHal sind bekannte oder Analoge bekannter Verbindungen, welche in bekannter oder an sich bekannter Weise hergestellt werden können (z.B. Houben-Weyl, Methoden der Organischen Chemie, Band 13/8: Metallorganische Verbindungen As, Sb, Bi; Georg Thieme Verlag Stuttgart, 1978).

Die erfindungsgemässen Komplexe mit Verbindungen der Formel I eignen sich beispielsweise für die asymmetrische Hydrierung von Chromenylpyridin-Derivaten der allgemeinen Formel IV zu Verbindungen der Formel V 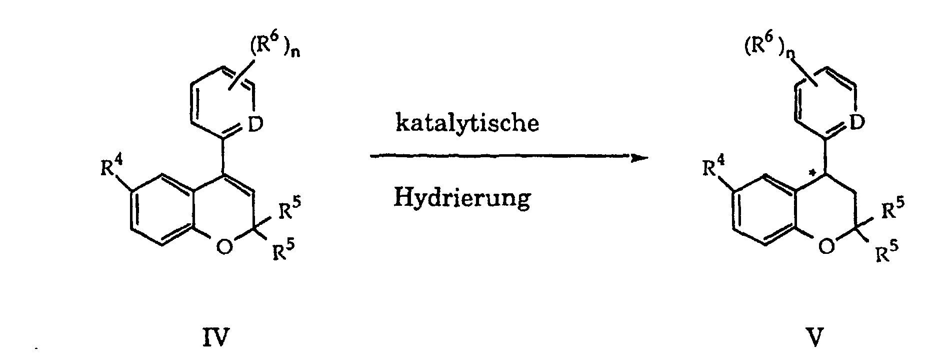 worin
- D: N- oder N-oxid
- R⁴: Wasserstoff, Cyano, Halogen, Nitro,Trifluormethyl, C₁₋₈-Alkyl, C₁₋₈-Alkoxycarbonyl, C₁₋₈-Alkylthio, C₁₋₈-Alkylsulfonyl, Aroyl, Carbamoyl, Mono( C₁₋₈-alkyl)carbamoyl oder Di( C₁₋₈-alkyl)carbamoyl, C₁₋₈-Alkanoyl
- R⁵: Wasserstoff, C₁₋₈-Alkyl oder CH₂F
- R⁶: C₁₋₈-Alkyl, Halogen, Amin, CO₂-(C₁₋₈-Alkyl), C₁₋₈-Alkoxy, Hydroxy,Phenyl, Tolyl oder im Fall von n = 2 ein Benzorest und
- n: 0, 1 oder 2 ist
in geeigneten, unter den Reaktionsbedingungen inerten organischen Lösemittel erfolgen. Als derartige Lösemittel werden insbesondere niedere Alkohole, halogenierten Kohlenwasserstoffen, oder Gemische obengenannter Lösemittel mit Ethern, oder Gemische von Alkoholen mit Estern, oder mit Ketonen verwendet.

"Aroyl" bedeutet gegebenenfalls durch ein Halogen oder Nitro substistuiertes Benzoyl und "Aryl" gegebenenfalls durch Halogen, Cyan oder C₁₋₈-Alkyl ein- oder mehrfach substituiertes Phenyl oder Naphthyl.

Bevorzugte Lösemittel für die Hydrierung der Chromenylpyridin-N-oxide sind Ester, Kohlenwasserstoffe, Ether oder Gemische davon.

Die asymmetrische Hydrierung von Verbindungen der Formel IV wird vorzugsweise für die Chromenylpyridin-N-oxide in Gegenwart von RhodiumKomplexen der Formeln

[Rh(Y)(Lₙ)]⁺A⁻ II-a

[Rh(Y)(Lₙ)B] II-b

durchgeführt.

Die asymmetrische Hydrierung von Verbindungen der Formel IV wird vorzugsweise für die Chromenylpyridine in Gegenwart von Ruthenium-Komplexen der Formeln

[Ru(Y)]²⁺(A⁻)₂ II-c

[Ru(Y)(B)₂] II-d

[Ru(Y)(C¹)(C²)₂₋ₘ](C³)ₘ II-e

durchgeführt

Für die Hydrierung der Chromenylpyridine eignen sich als Lösemittel vorzugsweise chlorierte Kohlenwasserstoffe, Alkohole oder Gemische davon.

Die Hydrierung wird zweckmässig bei Temperaturen im Bereich von etwa 0°C bis 150°C, vorzugsweise 10 bis 100°C, besonders bevorzugt im Temperaturbereich von etwa 20°C bis 80°C, und einem Druck von etwa 1 bis 200 bar, bevorzugt 1 bis 150 bar und besonders bevorzugt 10 bis 80 bar, durchgeführt.

Das molare Verhältnis Substrat / Katalysator (S/C) zwischen den zu hydrierenden Verbindungen der Formel III und dem Metallkomplexen, die als Katalysatoren gemäss der Formeln II-a bis II-e verwendet werden, liegt zweckmässig zwischen 20 bis 30000, vorzugsweise zwischen 100 bis 6000.

Die Komplexe aus einem Metall der Gruppe VIII und einer Verbindung der Formel I, wie z.B. die Komplexe der Formeln II-a bis II-e können analog zu entsprechenden Diphosphinliganden in an sich bekannter Weise hergestellt werden, beispielsweise wie in der EP 398 132 beschrieben.

Die nachfolgenden Beispiele dienen zur Illustrierung der Erfindung und sollen in keiner Weise eine Beschränkung darstellen. In den Beispielen haben die gewählten Ausdrücke die folgende Bedeutung:
- HPLC: Hochdruck-Flüssigkeitschromatographie
- e.e.: Enantiomeren Ueberschuss (Enantiomeric Excess)
- RT: Raumtemperatur
- Smp.: Schmelzpunkt
- (R)-BIPHAS: (R)-6,6'-Dimethyl-biphenyl-2,2'-diyl-bis(diphenylarsin)
- (S)-p-Tol-BIPHAS: (S)-6,6'-Dimethyl-biphenyl-2,2'diyl-bis(di-p-tolylarsin)
- (Rh(COD)₂BF₄: Bis-(Cycloocta-1,5-dien)rhodium(I)-tetrafluoroborat
Alle Temperaturen sind in ° Celsius angegeben.

### Beispiel 1

### (R)-(6,6'-Dimethylbiphenyl-2,2'-diyl)bis(diphenylarsin)

In einem 3L-Kolben mit Magnetrührer, Tropftrichter und Stickstoffbegasung wurden 91.8 g (0.40 mol) Diphenylarsin in 250 ml trockenem Tetrahydrofuran vorgelegt. Innerhalb von 15 min wurde innert unter Eisbadkühlung eine Lösung von 62 g (0.244 mol) Iod in 100 ml trockenem Tetrahydrofuran zugetropft. Nach einer zusätzlichen Rührdauer von 15 min wurde die Lösung eingedampft und der Rückstand in 50 ml trockenem Tetrahydrofuran aufgenommen.

In einem 2.5L-Sulfierkolben mit Magnetrührer, Intensivkühler, Tropftrichter, Thermometer und Stickstoffbegasung wurden 30.0 g (69.1 mmol) (R)-2,2'-Diiod-6,6'-dimethylbiphenyl in 600 ml trockenem Toluol und 100 ml Ether vorgelegt. Bei -70° bis -55° wurden innert 25 min 100 ml 1.6 N Butyllithiumlösung in Hexan (160 mmol) zugetropft, und die Mischung wurde noch 15 min bei -60° gerührt. Anschliessend wurde innert 15 min die oben erhaltene Tetrahydrofuranlösung von Ioddiphenylarsin bei -50° zugetropft. Nach Rühren über Nacht bei RT wurde mit 250 ml Wasser versetzt, 30 min gerührt und mit 500 ml Essigsäureäthylester verdünnt. Die organische Phase wurde abgetrennt, mit Wasser neutral gewaschen, über Na₂SO₄ getrocknet, filtriert und das Filtrat eingedampft. Der Rückstand (151.6 g) wurde an 500 g Kieselgel chromatographiert (Elution Hexan/Toluol 0% 10%). Es wurden 13.0 g (29%) (R) -(6,6'-Dimethylbiphenyl-2,2'-diyl)bis(diphenylarsin) als weisses Pulver isoliert. Zur Analyse wurde aus 10 ml Essigsäureäthylester umkristallisiert. Es wurden 7.8 g (17%) (R)-(6,6'-Dimethylbiphenyl-2,2'diyl)bis(diphenylarsin) vom Smp. 184-185° erhalten; [α]²⁰,_{D} = -108° (c = 1%, CHCl₃). Mikroanalyse: C₃₈H₃₂As₂ (638.5); ber.: C 71.48, H 5.05; gef.: C 71.67, H 5.38%.

### Beispiel 2

### (rac)-(6,6'-Dimethylbiphenyl-2,2'-diyl)bis(diphenylarsin)

wurde in zu Beispiel 1 analoger Weise aus (rac)-2,2'-Diiod-6,6'dimethylbiphenyl hergestellt; Smp. 209-210°.

### Beispiel 4

### (R)-(6,6'-Dimethylbiphenyl-2,2'-diyl)bis(dicyclohexylarsin)

In einem 1.5L-Sulfierkolben mit Thermometer, Tropftrichter und Intensivkühler wurden unter einer N₂-Atmosphäre 13.0 g (30.0 mmol) (R)-2,2'-Diiod-6,6'-dimethylbiphenyl in 300 ml trockenem Toluol und 50 ml Ether vorgelegt. Bei -70° wurden innert 15 min 44 ml 1.6 N Butyllithiumlösung in Hexan (70 mmol) zugetropft, und die Mischung wurde noch 30 min bei -70° gerührt. Dann wurden innert 20 min 38.7 g (120 mmol) Bromdicyclohexylarsin in 80 ml Toluol zugetropft. Nach Rühren bei 60° (2 Stunden) und bei RT über Nacht wurde eine Lösung von 2.60 g (102 mmol) Iod in 30 ml

Tetrahydrofüran zugetropft, 15 min gerührt, mit 100 ml Wasser und mit 150 ml IN Natronlauge versetzt und 30 min gerührt. Nach Zugabe von 500 ml Essigsäureäthylester wurde die organische Phase abgetrennt, mit Wasser neutralgewaschen, über Na₂SO₄ getrocknet, filtriert und das Filtrat eingedampft. Chromatographische Auftrennung des Rückstandes (32 g) an Kieselgel (600 g, Elution Hexan/Toluol 0% 20%) lieferte 8.0 g (40%) (R)-6,6'-Dimethylbiphenyl-2,2'-diyl)bis(dicyclohexylarsin). Die Probe zur Analyse wurde aus CH₂Cl₂/ Essigsäureäthylester 1:1 umkristallisiert; Smp. 199.5-200.5°; [α]²⁰,_{D} = -102.7° (c = 1%, CHCl₃). Mikroanalyse: C₃₈H₅₆As₂ (662.71); ber.: C 68.87, H 8.52; gef.: C 68.78, H 8.42%.

### Beispiel 6

### (S)-(6,6'-Dimethylbiphenyl-2,2'-diyl)bis(di-p-tolylarsin)

Eine in Analogie zu Beispiel 1 durchgeführte Reaktion von Di-ptolylarsin [hergestellt aus 42.5 g (122 mmol) Tri-p-tolylarsin] und 17.0 g (40 mmol) (S)-2,2'-Diiod-6,6'-dimethylbiphenyl lieferte 47.5 g eines Produktgemisches. Dieses wurde an 600 g Kieselgel chromatographiert (Elution Hexan/Toluol 0% 100%) und ergab 9.9 g (36%) (S)-(6,6'-Dimethylbiphenyl-2,2'-diyl)bis(di-p-tolylarsin). Die Probe zur Analyse wurde aus Essigester/Methanol umkristallisiert; Smp. 216-217°; [α]²⁰,_{D} = +107.9° (c = 1%, CHCl₃). Mikroanalyse: C₄₂H₄₀As₂ (694.63); ber.: C 72.62, H 5.80; gef.: C 72.79, H 5.96%.

### Beispiel 8

### (rac)-(6,6'-Dimethylbiphenyl-2,2'-diyl)bis(diphenylarsin)

Eine Lösung von 10.0 g (38 mmol) Diphenylarsinsäure in 50 ml Toluol und 50 ml Thionylchlorid wurde 4 h unter Rückfluss gekocht. Nach Abkühlung wurde die Reaktionslösung eingedampft und der Rückstand in 50 ml Toluol aufgenommen.

In einem 750 ml-Sulfierkolben mit Magnetrührer, Kühler, Tropftrichter, Thermometer und N2-Begasung wurden 5.2 g (12 mmol) (R,S)-2,2'-Diiod-6,6'dimethylbiphenyl in 80 ml Toluol und 20 ml Äther vorgelegt. Bei -70° wurden innert 3 min 17 ml 1.6 N Butyllithiumlösung in Hexan (27 mmol) zugetropft, und es wurde noch eine Stunde bei -70° gerührt. Anschliessend wurde die oben erhaltene Toluollösung bei -70° zugetropft. Nach Rühren über Nacht bei Raumtemperatur wurde die Lösung mit 100 ml Wasser und 10 ml 3 N NaOH versetzt, eine Stunde gerührt, die Phasen getrennt und die wässrige Phase mit 500 ml Toluol extrahiert. Die vereinigten organischen Phasen wurden mit Wasser neutralgewaschen, über Na₂SO₄ getrocknet, filtriert und das Filtrat eingedampft. Der Rückstand (5.8 g) wurde an 300 g Kieselgel chromatographiert. Mit Hexan/Toluol 0% 20% wurden 1.0 g (13%) (rac)-(6,6'-Dimethylbiphenyl-2,2'-diyl)bis(diphenylarsin) eluiert. Eine Probe zur Analyse wurde aus Essigsäureäthylester umkristallisiert; Mikroanalyse: C₃₈H₃₂As (638.52); ber.: C 71.48, H 5.05; gef.: C 71.06, H 5.24%.

### Beispiel 9

### (R)-2-(6-Cyano-3,4-dihydro-2,2-dimethyl-2H-1-benzopyran-4-yl)pyridin-1-oxid

In einer Glove-Box (O₂-Gehalt < 1 ppm) wurden 100 mg (359 mMol) 2-(6-Cyano-2,2-dimethyl-2H-1-benzopyran-4-yl)pyridin-1-oxid, 9 ml Toluol, 1 ml Dichlormethan, 5.8 mg (14,4 mMol) [Rh(COD)₂]BF₄ und 6.1 mg (14,4 mMol) (R)-BIPHAS in einem 30 ml-Autoklav vorgelegt. Der Autoklav wurde verschlossen und die Hydrierung unter Rühren bei 40° und einem Druck von 40 bar durchgeführt. Die Hydrierung wurde nach 20 h abgebrochen. Zur Bestimmung des e.e.-Wertes und des Umsatzes wurde eine Probe der Hydrierlösung eingedampft und durch HPLC an einer chiralen Phase (Chiralcel OD-H) analysiert. Man erhielt (R)-2-(6-Cyano-3,4-dihydro-2,2-dimethyl-2H-1-benzopyran-4-yl)pyridin-1-oxid in quantitativer Ausbeute: e.e. = 85%; chem. Reinheit >99%.

### Beispiel 10

### (S)-2-(6-Cyano-3,4-dihydro-2,2-dimethyl-2H-1-benzopyran-4-yl)pyridin-1-oxid

In zu Beispiel 9 analoger Weise wurde die Hydrierung mit (S)-p-Tol-BIPHAS durchgeführt. Man erhielt (S)-2-(6-Cyano-3,4-dihydro-2,2-dimethyl-2H-1-benzopyran-4-yl)pyridin-1-oxid in quantitativer Ausbeute: e.e. = 88%; chem. Reinheit >99%.

## Patentansprüche

1. Chirale, in der (R)-oder (S)-Form oder als Racemat vorliegende Diarsinverbindungen der allgemeinen Formel 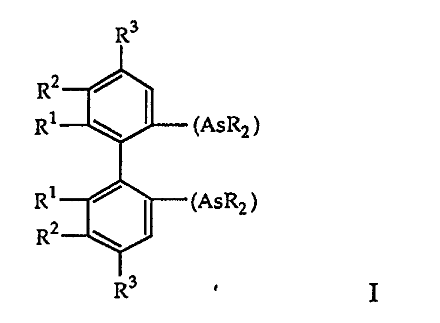 worin
R ein gegebenenfalls substituiertes Aryl aus der Gruppe von Phenyl, Naphthyl, Furyl und Thienyl; C₃₋₈-Cycloalkyl oder C₁₋₈-Alkyl;
R¹ Methyl,
R², R³ jeweils unabhängig voneinander C₁₋₈-Alkyl, C₁₋₈-Alkoxy, Aryloxy, F oder Cl,
R² und R³ unabhängig voneinander auch Wasserstoff oder
R¹ und R² zusammen Tetramethylen, Dioxomethylen oder ein Benzo- oder Benzofuro- System an dem jeweiligen Benzolring bedeuten.

2. (R)- oder (S)-(6,6'-Dimethylbiphenyl-2,2'-diyl)-bis-(diphenylarsin).

3. (R)- oder (S)-(6,6'-Dimethylbiphenyl-2,2'-diyl)-bis-(di-p-tolylarsin).

4. (R)- oder (S)-(6,6'-Dimethylbiphenyl-2,2'-diyl)-bis-(dicyclohexylarsin).

5. Verfahren zur Herstellung von chiralen, in (R)- oder (S)-oder (rac)-Form vorliegenden Diarsinverbindungen der allgemeinen Formel 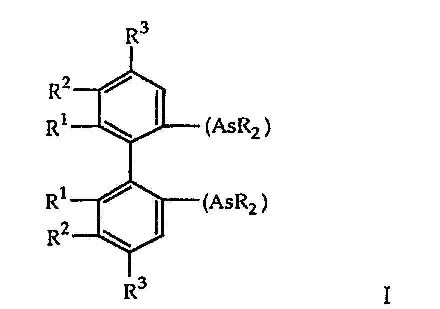 worin R, R1, R2 und R3 die Bedeutung gemäss Anspruch 1 hat **dadurch gekennzeichnet, dass** man eine Verbindung der allgemeinen Formel 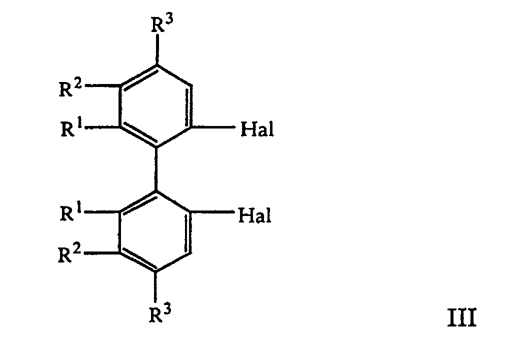 worin R, R¹, R² und R³ die obige Bedeutung haben,
mit einer Verbindung der Formel R₂AsHal, worin R die Bedeutung gemäss Anspruchs 1 hat und Hal Brom oder Iod darstellt, in Gegenwart einer Alkalialkyl oder Alkali-aryl-Verbindung umsetzt.

6. Komplexe von Diarsinverbindungen der Formel I gemäss einem der Ansprüche 1 bis 4 mit einem Metall der Gruppe VIII.

7. Komplexe gemäss Anspruch 6, worin als Metall der Gruppe VIII Ruthenium oder Rhodium enthalten sind.

8. Verwendung der chiralen in der (R)- oder (S)-Form oder als Racemat vorliegenden Diarsinverbindungen der Formel I
worin
R ein gegebenenfalls substituiertes Aryl aus der Gruppe von Phenyl, Naphthyl, Furyl und Thienyl; C₃₋₈-Cycloalkyl oder C₁₋₈-Alkyl;
R¹ R², R³, jeweils unabhängig voneinander C₁₋₈-Alkyl, C₁₋₈-Alkoxy, Aryloxy, F oder Cl,
R² und R³ unabhängig voneinander auch Wasserstoff oder
R¹ und R² zusammen Tetramethylen, Dioxomethylen oder ein Benzofuro-System an dem jeweiligen Benzolring bedeuten,
in Form ihrer Komplexe mit einem Metall der Gruppe VIII, als Katalysatoren bei der asymmetrischen Hydrierung von Chromenylpyridin-N-oxiden und Chromenylpyridinen.

9. Verwendung der Diarsinverbindungen der Formel I gemäss Anspruch 1, in Form ihrer Komplexe mit einem Metall der Gruppe VIII, als Katalysatoren bei asymmetrischen Hydrierungen und für enantioselektive Wasserstoffverschiebungen.

## Revendications

1. Diarsines chirales, se présentant sous forme (R) ou (S) ou sous forme de racémates, de formule générale 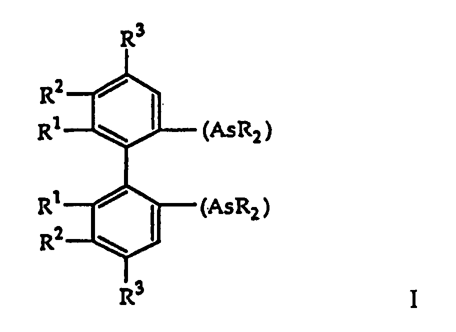 dans laquelle
R représente un reste aryle du groupe des restes phényle, naphtyle, furyle et thiényle, éventuellement substitué; un reste cycloalkyle en C₃-C₈ ou un reste alkyle en C₁-C₈;
R¹ représente un reste méthyle;
R², R³ représentent chacun, indépendamment l'un de l'autre, un reste alkyle en C₁-C₈, alcoxy en C₁-C₈, aryloxy, F ou Cl;
R² et R³, indépendamment l'un de l'autre, représentent aussi l'hydrogène ou
R¹ et R² forment ensemble un reste tétraméthylène ou dioxométhylène ou un système benzo ou benzofuro sur le cycle benzène correspondant.

2. (R)- ou (S)-(6,6'-diméthylbiphényl-2,2'-diyl)-bis(diphénylarsine).

3. (R)- ou (S)-(6,6'-diméthylbiphényl-2,2'-diyl)-bis(di-p-tolylarsine).

4. (R)- ou (S)-(6,6'-diméthylbiphényl-2,2'-diyl)-bis(dicyclohexylarsine).

5. Procédé de préparation de diarsines chirales, se présentant sous forme (R) ou (S) ou racémique, de formule générale 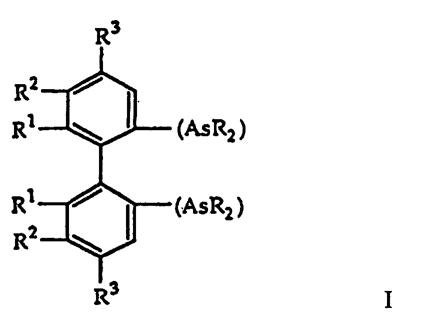 dans laquelle R, R¹, R² et R³ ont la signification indiquée dans la revendication 1, **caractérisé en ce que** l'on fait réagir un composé de formule générale 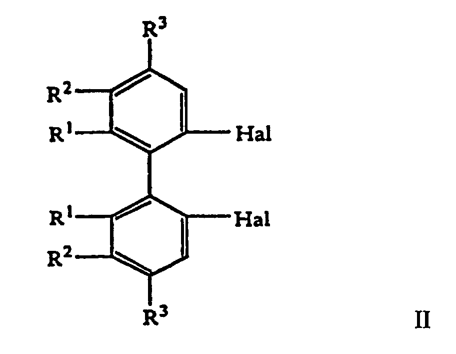 dans laquelle R, R¹, R² et R³ ont la signification indiquée ci-dessus,
avec un composé de formule R₂AsHal, dans laquelle R a la signification indiquée dans la revendication 1 et Hal représente le brome ou l'iode, en présence d'un composé d'alkyl(métal alcalin) ou d'aryl(métal alcalin).

6. Complexes de diarsines de formule I selon l'une des revendications 1 à 4 avec un métal du groupe VIII.

7. Complexes selon la revendication 6, contenant comme métal du groupe VIII du ruthénium ou du rhodium.

8. Utilisation des diarsines chirales de formule I, se présentant sous forme (R) ou (S) ou sous forme de racémates, dans lesquelles
R représente un reste aryle du groupe des restes phényle, naphtyle, furyle et thiényle, éventuellement substitué; un reste cycloalkyle en C₃-C₈ ou un reste alkyle en C₁-C₈;
R¹, R², R³ représentent chacun, indépendamment les uns des autres, un reste alkyle en C₁-C₈, alcoxy en C₁-C₈, aryloxy, F ou Cl;
R² et R³, indépendamment l'un de l'autre, représentent aussi l'hydrogène ou
R¹ et R² forment ensemble un reste tétraméthylène ou dioxométhylène ou un système benzofuro sur le cycle benzène correspondant,
sous forme de leurs complexes avec un métal du groupe VIII, comme catalyseurs dans l'hydrogénation asymétrique de chroménylpyridine-N-oxydes et de chroménylpyridines.

9. Utilisation des diarsines de formule I selon la revendication 1, sous forme de leurs complexes avec un métal du groupe VIII, comme catalyseurs dans des hydrogénations asymétriques et pour des déplacements d'hydrogène énantiosélectifs.

## Claims

1. Chiral diarsine compounds, which are present in the (R)- or (S)-form or as a racemate, of the general formula 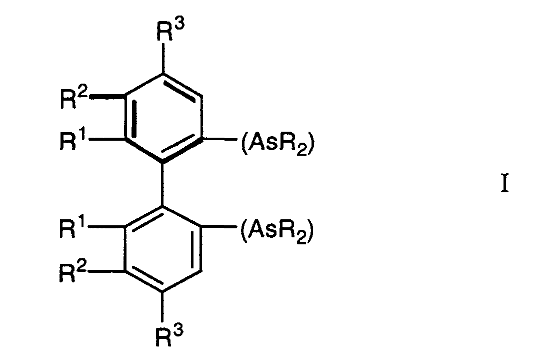 wherein
R signifies an optionally substituted aryl from the group of phenyl, naphthyl, furyl and thienyl; C₃₋₈-cycloalkyl or C₁₋₈-alkyl;
R¹ signifies methyl,
R², R³ each independently signify C₁₋₈-alkyl, C₁₋₈-alkoxy, aryloxy, F or Cl,
R² and R³ each independently also signify hydrogen or
R¹ and R² together signify tetramethylene, dioxomethylene or a benzo or benzofuro system on the respective benzene ring.

2. (R)- or (S)-(6,6'-Dimethylbiphenyl-2,2'-diyl)-bis-(diphenylarsine).

3. (R)- or (S)-(6,6'-Dimethylbiphenyl-2,2'-diyl)-bis-(di-p-tolylarsine).

4. (R)- or (S)-(6,6'-Dimethylbiphenyl-2,2'-diyl)-bis-(dicyclohexylarsine).

5. A process for the manufacture of chiral diarsine compounds, which are present in the (R)- or (S)-form or as a racemate, of the general formula 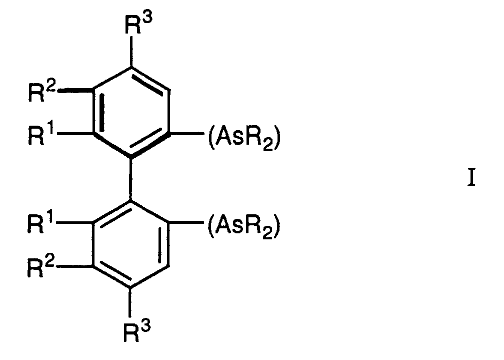 wherein R, R1, R2 and R3 has the significance according to claim 1,
**characterized by** reacting a compound of the general formula 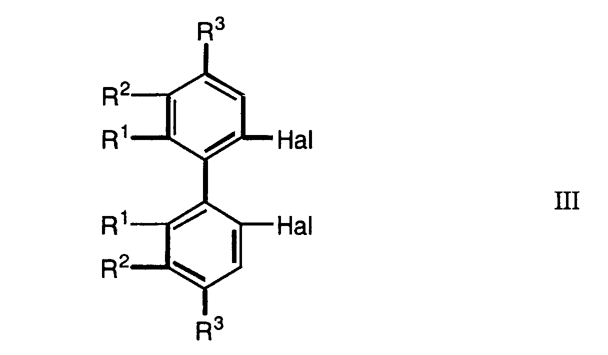 wherein R, R¹, R² and R³ have the above significance,
with a compound of the formula R₂AsHal, wherein R has the significance according to claim 1 and Hal represents bromine or iodine, in the presence of an alkali-alkyl or alkali-aryl compound.

6. Complexes of diarsine compounds of formula I according to any one of claims 1 to 4 with a Group VIII metal.

7. Complexes according to claim 6, wherein ruthenium or rhodium is present as the Group VIII metal.

8. The use of chiral diarsine compounds of formula I, which are present in the (R)-or (S)-form or as a racemate,
wherein
R signifies an optionally substituted aryl from the group of phenyl, naphthyl, furyl and thienyl; C₃₋₈-cycloalkyl or C₁₋₈-alkyl;
R¹, R², R³, each independently signify C₁₋₈-alkyl, C₁₋₈-alkoxy, aryloxy, F or Cl,
R² and R³ each independently also signify hydrogen or
R¹ and R² together signify tetramethylene, dioxomethylene or a benzofuro system on the respective benzene ring,
in the form of their complexes with a Group VIII metal as catalysts in the asymmetric hydrogenation of chromenylpyridine N-oxides and chromenylpyridines.

9. The use of the diarsine compounds of formula I according to claim 1 in the form of their complexes with a Group VIII metal as catalysts in asymmetric hydrogenations and for enantioselective hydrogen displacements.
